# EUROPÄISCHE PATENTSCHRIFT

(11) **EP 1 863 908 B1**
(45) Veröffentlichungstag und Bekanntmachung des Hinweises auf die Patenterteilung: **17.11.2010**
(21) Anmeldenummer: 06723749.5
(22) Anmeldetag: 27.03.2006
(51) Int. Cl.: C12N 9/22, C12Q 1/68

(54) **REVERSE TRANSKRIPTION UND AMPLIFIKATION VON RNA BEI SIMULTANER DEGRADIERUNG VON DNA**
REVERSE TRANSCRIPTION AND AMPLIFICATION OF RNA WITH SIMULTANEOUS DEGRADATION OF DNA
TRANSCRIPTION INVERSE ET AMPLIFICATION D'ARN AVEC DEGRADATION SIMULTANEE D'ADN

(30) Priorität: 01.04.2005 EP 05007157
(43) Veröffentlichungstag der Anmeldung: 12.12.2007
(73) Patentinhaber: Qiagen GmbH, 40724 Hilden (DE)
(72) Erfinder: KORFHAGE, Christian, 40764 Langenfeld (DE); PEIST, Ralf, 40723 Hilden (DE); LÖFFERT, Dirk, 40589 Düsseldorf (DE)
(86) Internationale Anmeldenummer: PCT/EP2006/002771
(87) Internationale Veröffentlichungsnummer: WO 2006/103039

(56) Entgegenhaltungen:
- EP-A- 0 278 340
- EP-A- 1 132 470
- US-A1- 2002 172 972
- KYLE M ET AL: "A microfluorometric method for quantifying RNA and DNA in terrestrial insects." JOURNAL OF INSECT SCIENCE (TUCSON), Bd. 3, Nr. 1 Cited January 10, 2003, 7. Januar 2003 (2003-01-07), Seiten 1-7 URL, XP002390171 ISSN: 1536-2442
- SANYAL A ET AL: "An effective method of completely removing contaminating genomic DNA from an RNA sample to be used for PCR" MOLECULAR BIOTECHNOLOGY, TOTOWA, NJ, US, Bd. 8, August 1997 (1997-08), Seiten 135-137, XP002092903 ISSN: 1073-6085
- BAUER P ET AL: "Use of manganese in RT-PCR eliminates PCR artifacts resulting from DNase I digestion" BIOTECHNIQUES, Bd. 22, Nr. 6, 1997, Seiten 1128-1130, 1132, XP002327922 ISSN: 0736-6205
- "Avoiding DNA contamination in RT-PCR" INTERNET ARTICLE, [Online] 3. Oktober 2004 (2004-10-03), XP002327921 Gefunden im Internet: URL:http://www.ambion.com/techlib/tb/tb_17 6.html> [gefunden am 2005-05-11]
- "Methods to remove DNA contamination from RNA samples" INTERNET ARTICLE, [Online] 30. Oktober 2004 (2004-10-30), XP002327967 Gefunden im Internet: URL:www.ambion.com/techlib/tb/tb_181.html> [gefunden am 2005-05-12]
- "Stratagene Catalog: RT-PCR Systems and Kits" STRATAGENE CATALOGUE, 1999, Seiten 154-155, XP002264374

## Beschreibung

Die vorliegende Erfindung betrifft ein Verfahren zur Behandlung von RNA, insbesondere ein RNA-Reaktionsverfahren.

Viele Techniken in der Molekularbiologie führen zur Analyse von Ribonukleinsäuren (RNA). Um RNA analysieren zu können, muss diese zuvor von allen inhibitorischen und kontaminierenden Substanzen gereinigt werden. So können z.B. Kontaminationen von genomischer Desoxyribonukleinsäure (DNA) inhibitorisch wirken oder zu falsch positiven Ergebnissen führen. Einige Techniken molekularer RNA-Analytik starten mit der reversen Transkription von RNA in cDNA. cDNA ist in Struktur und Sequenz sehr ähnlich bis identisch zu genomischer DNA. Daher kann eine Kontamination von genomischer DNA zu falschen Ergebnissen führen, wenn cDNA analysiert werden soll (z.B. photometrische Bestimmung der cDNA-Menge oder deren Quantifizierung durch PCR).

Um RNA sicher analysieren zu können, ist es daher notwendig, alle störenden anderen Nukleinsäuren, wie z.B. genomische DNA, vor einer entsprechenden Analyse entweder von der RNA abzutrennen oder in ihre einzelnen Bausteine zu zerlegen. Ein seit langem angewandtes Trennungsverfahren für DNA und RNA ist die sog. Dichtegradientenzentrifugation. Standardsubstanzen für die Dichtegradientenzentrifugation sind Cäsiumchlorid (CsCl) und Saccharose. Für CsCl stellt sich z.B. je nach Dichte der Ausgangslösung während der Zentrifugation im Gleichgewichtszustand ein Dichtegradient ein, in dem sich jedes Makromolekül in der seiner eigenen Dichte entsprechenden Zone im Gradienten einordnet. Um die sich nach der Zentrifugation einstellenden Nukleinsäurebanden in dem Zentrifugationsgefäß sichtbar zu machen, wird der CsCI-Lösung Ethidiumbromid zugegeben, das sich in die Nukleinsäure einlagert und im UV-Licht fluoresziert. Mit dieser Methode lassen sich einzelne DNA-Bruchstücke, die aufgrund der eng zusammenliegenden Sedimentationsraten dieser Bruchstücke ansonsten sehr schwer voneinander trennbar sind, sicher abtrennen. Bei der CsCl-Dichtegradientenzentrifugation verwendet man üblicherweise Gradienten mit Dichtewerten zwischen 1,0 und 1,9 g/ml. Da die Schwebedichte der RNA üblicherweise größer als 1,9 g/ml ist, schlägt sich die RNA bei einer Gleichgewichtszentrifugation (auch isopyknische Zentrifugation genannt) in einem Gradienten, dessen Dichte-Obergrenze bei 1,9 g/ml liegt, am Boden des Probengefäßes nieder, während alle anderen Molekülarten (und damit auch die DNA) jeweils Banden innerhalb des Gradienten ausbilden. Entsprechende Trennungsoperationen führen daher zu einer guten Auftrennung von RNA und DNA. Das Dichtegradientenzentrifugations-Verfahren ist jedoch aufgrund der eingesetzten Chemikalien relativ teuer, apparativ sehr aufwendig und auch sehr zeitintensiv (bis sich ein Gleichgewicht eines üblichen CsCl-Gradienten eingestellt hat, sind mit den meisten Rotoren lange Zentrifugenläufe von bis zu 2 Tagen notwendig).

Deshalb wurde bei der Gewinnung von RNA dazu übergegangen, während oder nach der RNA-Präparation dem Versuchsansatz eine DNase oder mehrere DNasen zuzugeben, um DNA Kontaminationen enzymatisch abzubauen. Auf dem Markt erhältliche Systeme (sog. "Kits") für diesen Zweck werden von der Firma QIAGEN, Hilden, Deutschland unter den Bezeichnungen *"RNeasy Micro Kit"* und *"RNeasy Fibrous Tissue Kit"* und von der Firma Promega, Madison/WI, USA, unter der Bezeichnung *"SV Total RNA Isolation System"* angeboten. Allerdings führen diese RNA-Präparationsmethoden nicht zur Isolierung reiner RNA. Die gewonnene RNA liegt vielmehr als Material vor, das noch in unterschiedlichem Maße mit genomischer DNA, Salzen, Inhibitoren etc. verunreinigt ist. Für manche Anwendungen mag der mit den o.g. Kits erreichbare Reinigungsgrad ausreichend sein, für einige andere Einsatzgebiete (z.B. RT-PCR) ist dies jedoch nicht der Fall.

Auch wird noch ein Verfahren zur RNA-Reinigung eingesetzt, bei dem chromatographische Methoden (z.B. Ionenaustausch-Chromatographie, Oligo-dT-Chromatographie) verwendet werden, um RNA nochmals anzureichern und die Menge an DNA zu reduzieren. Mit dieser Methode ist es allerdings nicht möglich, ribosomale RNA aufzureinigen.

Schließlich beschreibt die US-Patentanmeldung Nr. 20020042052 noch ein Verfahren zur Entfernung von Nukleinsäureverunreinigungen aus einem Ansatz für Amplifikationsreaktionen. Dabei wird eine thermolabile DNase eingesetzt, die immer vor der eigentlichen Amplifikationsreaktion unerwünschte doppelsträngige DNA in dem Amplifikationsansatz abbaut. Aufgrund ihrer Thermolabilität wird die eingesetzte DNase spätestens bei der ersten Temperaturerhöhung auf über 90°C bei der PCR-Reaktion irreversibel inaktiviert. Die PCR-Reaktion kann daher erst dann begonnen werden, wenn die DNase-Reaktion abgeschlossen ist. Eine Gleichzeitigkeit der DNA-Abbaureaktion und einer RNA-Reaktion ist daher bei dem aus der genannten amerikanischen Offenlegungsschrift bekannten Verfahren nicht gegeben.

Auch alle diese neueren Verfahren sind jedoch teilweise zeitaufwendig, kostenintensiv und können gegebenenfalls bei gleichzeitiger Bearbeitung mehrerer RNA-Präparationen zu Kreuzkontaminationen führen. Auch gilt für alle der oben beschriebenen vorbekannten Verfahren, dass dabei nicht simultan mit der RNA-Reaktion oder RNA-Analyse eine DNA-Degradierung erfolgt, sondern immer vor der eigentlichen RNA-Reaktion oder RNA-Analyse der DNA Abbau durchgeführt wird.

Der vorliegenden Erfindung liegt somit die Aufgabe zugrunde, ein Verfahren für RNA-Reaktionen zur Verfügung zu stellen, das die Nachteile der oben beschriebenen bekannten Verfahren nicht aufweist. Das neue Verfahren soll kostengünstig und wenig zeitaufwendig sein und den apparativen Aufwand in Grenzen halten.

Diese Aufgabe löst die Erfindung durch das Verfahren gemäß dem unabhängigen Anspruch 1. Weitere vorteilhafte Ausgestaltungen, Aspekte und Details der Erfindung ergeben sich aus den abhängigen Ansprüchen, der Beschreibung, den Beispielen und den Figuren.

Die vorliegende Erfindung betrifft somit ein RNA-Reaktionsverfahren, das dadurch gekennzeichnet ist, dass im selben Reaktionsgefäß eine RNA-Reaktion und eine Degradierung von vorhandener doppelsträngiger DNA erfolgen, wobei die Degradierung der doppelsträngigen DNA durch DNase I erfolgt. Die RNA-Reaktion und die Degradierung vorhandener doppelsträngiger DNA finden gleichzeitig statt. Dies hat den großen Vorteil, dass mit dem Beginn einer RNA-Reaktion nicht mehr gewartet werden muss, bis die in dem Reaktionsansatz vorhandene, unerwünschte doppelsträngige DNA vollständig oder zumindest soweit abgebaut ist, dass sie die RNA-Reaktion oder die anhängige Analysereaktion nicht mehr stört. Auch wird durch das erfindungsgemäße Verfahren die Gefahr vermindert, durch häufiges Öffnen des Reaktionsgefäßes Verunreinigungen in den Reaktionsansatz einzubringen.

Auch können bei dem erfindungsgemäßen Verfahren die RNA-Reaktion einerseits und der DNA-Abbau bei derselben Temperatur erfolgen. Die Temperatur kann dabei z.B. in einem Bereich von 10 bis 80°C, vorzugsweise 20 bis 70°C, insbesondere 20 bis 60°C, liegen.

Die Erfindung verknüpft somit erstmals die DNA-Dekontaminierung einer Probe mit der RNA-Reaktion in einem simultanen Prozess, d.h. die DNA-Dekontaminierung einerseits und die RNA-Reaktion bzw. die RNA-Analyse andererseits laufen gleichzeitig bzw. nebeneinander in ein und demselben Reaktionsgefäß ab. Typische RNA-Reaktionen sind z.B. reverse Transkription oder 1-Step-RT-PCR (Reverse Transkription Polymerasekettenreaktion in einem Schritt).

Die vorliegende Erfindung gewährleistet somit eine Degradierung (d.h. den Abbau) unerwünschter doppelsträngiger DNA (z.B. genomischer DNA (gDNA), linearer oder zirkulärer DNA, z.B. Plasmid-DNA) gleichzeitig mit Reaktionen, die RNA als Matrize enthalten (z.B. reverse Transkription, siehe oben). Die Degradierung der doppelsträngigen DNA erfolgt innerhalb der Reaktion, die RNA als Matrize enthält, durch DNase I. Mit der vorliegenden Erfindung ist es erstmals möglich, dass in Gegenwart z.B. einer cDNA-Synthese-Reaktion, bei der eine RNA in einzelsträngige DNA umgeschrieben wird, simultan DNase I zur Degradierung doppelsträngiger DNA eingesetzt wird, wobei die gerade im Syntheseprozess entstehende, einzelsträngige cDNA nur sehr wenig oder gar nicht degradiert wird.

Wie oben bereits erwähnt, betrifft die vorliegende Erfindung die Verknüpfung von Verringerung der dsDNA-Kontamination einerseits und RNA-Reaktion andererseits in einem simultanen Prozess. Wichtig dabei sind die Reaktionsbedingungen, die gleichermaßen eine DNA-Degradierung wie auch die Reaktion mit der RNA erlauben, wobei die dsDNA-Dekontaminierung mittels DNase I durchgeführt wird.

Eine "DNA-Kontamination" oder "DNA-Verunreinigung" in einem RNA-Isolat ist definiert als jegliches doppelsträngige Desoxyribonukleinsäure-Molekül, das unterschiedlicher Herkunft sein kann und zusammen mit der RNA im selben Reaktionsgefäß als unerwünschtes Molekül vorkommt. Doppelsträngigkeit der DNA kann auch dadurch entstehen, dass eine Einzelstrang-DNA durch Selbst-Hybridisierung zurückgefaltet vorliegt und somit zumindest zeitweise doppelsträngig vorkommt.

Die doppelsträngige DNA (dsDNA) kann dem ursprünglichen biologischen Material entstammen, aus dem auch die RNA isoliert worden ist. Somit kann diese nukleärer, plastidärer oder mitochondrialer Natur sein. Die dsDNA kann auch aus einer externen Quelle auf biologischem Wege auf das ursprüngliche biologische Material übertragen worden sein, sei es durch Infektion, Transformation, Fusion, Inkorporation oder ähnliches und kann somit z.B. viraler, prokaryontischer oder eukaryontischer Herkunft sein. Zudem kann die DNA auch auf unnatürlichem Wege auf das ursprüngliche biologische Material übertragen worden sein, wie z.B. durch Elektroporation, Transformation, Transfektion oder andere Techniken. Es kann sich dabei um genomische DNA, Plasmid-DNA, doppelsträngige Oligonukleotide (wie z.B. Primer-Dimere) oder andere Formen doppelsträngiger DNA handeln. Darüber hinaus kann die doppelsträngige DNA auch während oder nach der RNA-Isolierung in die RNA-Präparation eingeschleppt worden sein.

Als "RNA-Reaktion" wird z.B. definiert:
jede Form von Umsetzung, bei der RNA als Matrize für Polymerase-Reaktionen verwendet wird, wie z.B. (a) reverse Transkription oder (b) Transkription durch RNA-Polymerasen oder ähnliches;
zu (a): Reverse Transkription kann dabei durchgeführt werden durch mutierte und durch nicht mutierte RNA-abhängige DNA-Polymerasen, wie z.B. Reverse Transkriptasen von Viren, Retrotransposons, Bakterien etc. Diese können eine RNase H-Aktivität aufweisen oder es können Reverse Transkriptasen Verwendung finden, die so mutiert sind, dass die RNase H-Aktivität der Reversen Transkriptase eingeschränkt wurde oder nicht vorhanden ist (z.B. MMLV-RT RNase H⁻). RNA-abhängige DNA-Synthese (reverse Transkription) kann auch durchgeführt werden durch Enzyme, die durch Mutation oder veränderte Reaktionsbedingungen eine veränderte Nukleinsäure-Abhängigkeit aufweisen und so die Funktion der RNA-abhängigen DNA-Polymerase erhalten. Als Beispiel sei hier die Tth-DNA-Polymerase genannt, die DNA-abhängig ist und durch Verwendung veränderter Reaktionsbedingungen auch RNA als Matrize verwenden kann.
zu (b): Eine RNA-Polymerase Reaktionen ausgehend von RNA als Matrize kann durchgeführt werden durch mutierte wie durch nicht mutierte RNA-abhängige RNA-Polymerasen von z.B. Viren, Prokaryonten oder Eukaryonten. RNA-abhängige RNA-Synthese kann auch durchgeführt werden durch Enzyme, die durch Mutation oder veränderte Reaktionsbedingungen eine veränderte Nukleinsäure-Abhängigkeit aufweisen und so die Funktion der RNA-abhängigen RNA-Polymerase erhalten. Als Beispiel sei hier ein RNA-Amplifikationsverfahren, das eine T7-RNA Polymerase und RNA als Matrize verwendet, genannt (EP 1 056 884);

Wenn in Zusammenhang mit der vorliegenden Erfindung von "gleichzeitig" oder "Gleichzeitigkeit", "nebeneinander", "simultan" o.ä gesprochen wird, so ist darunter zu verstehen, dass eine dsDNA-Degradierung und die RNA-Reaktion im selben Reaktionsgefäß erfolgen. Eine Degradierung der dsDNA-Kontamination und die RNA-Reaktion werden daher zur selben Zeit und in ein und demselben Reaktionsansatz durchgeführt. Durch die Gleichzeitigkeit soll zum Ausdruck gebracht werden, dass die RNA-Reaktion und eine Degradierung der dsDNA-Kontamination zeitgleich im selben Reaktionsgefäß und unter denselben Reaktionsbedingungen ablaufen.

Vorteilhaft bei dem erfindungsgemäßen Verfahren, insbesondere gegenüber dem aus der US 20020042052 bekannten Verfahren, ist auch, dass das erfindungsgemäße Verfahren bei einer einheitlichen Temperatur ablaufen kann, d.h., dass die RNA-Reaktion und eine dsDN A-Degradierung bei der selben Temperatur ablaufen können. Außerdem ist es gegenüber dem aus der US 20020042052 bekannten Verfahren günstig, dass es bei dem erfindungsgemäßen Verfahren nicht notwendig ist, nach dem dsDNA-Abbau mittels DNase I das Reaktionsgefäß nochmals zu öffnen und ein neues Enzym (das bei einer Erwärmung auf über 90°C möglicherweise irreversibel geschädigt werden würde) zuzugeben, da dadurch unnötige Kontaminationen vermieden werden können.

Gemäß der vorliegenden Erfindung ist die RNA-Reaktion begrenzt durch die Reaktionsbedingungen, die nicht nur die RNA-Reaktion bestimmen, sondern auch simultan eine Degradierung der dsDNA-Kontamination ermöglichen sollen. Dies heißt nicht, dass für die Reaktion der RNA und eine Degradierung der dsDNA die jeweils optimalen Reaktionsbedingungen eingesetzt werden, sondern dass die Bedingungen für die RNA-Reaktion und eine Degradierung der DNA durchaus aneinander angepasst werden können. Die vorliegende Erfindung ermöglicht es erstmals, dass eine RNA-Reaktion und eine Degradierung von dsDNA in ein und demselben Reaktionsgefäß zeitgleich ablaufen können.

Unter einem "Abbau" bzw. einer "Degradierung" oder "Degradation" der dsDNA ist zu verstehen, dass der Abbauvorgang in jedem Fall so weit voranschreitet, dass die DNA nur noch geringe oder keine störenden Einflüsse mehr auf die RNA-Reaktion(en) oder die Nachfolge-Anwendungen zeigt. Ein Abbau bzw. eine Degradierung kann, muss aber nicht eine vollständige Zerlegung doppelsträngiger DNA in deren Einzelbausteine (Nukleotide) bedeuten. Die Degradierung der dsDNA erfolgt im Sinne der Erfindung zumindest teilweise gleichzeitig mit der RNA-Reaktion.

In der Zeichnung zeigen:
Fig. 1 ein Balkendiagramm, aus welchem die Ergebnisse eines Versuchs zur möglichen Beeinflussung der Reverse Transkriptase-Reaktion durch DNase I ersichtlich sind (Beispiel 1);
Fig. 2 ein Foto eines Agarose-Gels, welches das Ergebnis einer Elektrophorese gemäß Beispiel 2 zeigt;
Fig. 3 ein Diagramm, das die in Tabelle 1 genannten Ergebnisse in graphischer Form zeigt; und
Fig. 4 ein Balkendiagramm, das den Einfluss pankreatischer DNase I auf ein cDNA- und gDNA-Signal gemäß Beispiel 6 zeigt.

DNase I wird üblicherweise derart eingesetzt, dass der Reaktionsansatz etwa 0,01 bis etwa 100 U an Enzymaktivität enthält, vorzugsweise etwa 0,05 bis etwa 20 U, mehr bevorzugt etwa 0,1 bis etwa 10 U. Nach internationaler Übereinkunft entspricht die als 1 U (Unit, Enzymeinheit) angegebene Enzymaktivität a) für sequenzunspezifische DNA-Doppelstrang-spezifische Endonukleasen der Menge an Enzym, die benötigt wird, um pro Minute bei 25°C unter optimalen Bedingungen 1 µmol Substrat umzusetzen.

Die Degradierung der dsDNA wird im Falle einer RT-PCR in Anwesenheit einer Reversen Transkriptase durchgeführt. Taugliche Reverse Transkriptasen sind beispielsweise Reverse Transkriptasen aus Retroviren, wie z. B. HIV, AMV, MMLV, Omniscript^{®} (QIAGEN GmbH), Sensiscript^{®} (QIAGEN GmbH) etc. oder auch aus Retrotransposons. Die Reversen Transkriptasen können in ihrer Aminosäuresequenz dem Ursprungsorganismus entsprechen oder sie können auch Abweichungen davon enthalten, wie z.B. Veränderungen, die zum Verlust der RNase H-Aktivität führen, die Prozessivität verändern oder die Thermostabilität des Enzyms beeinflussen. Es können auch DNA-Polymerasen verwendet werden, die ursprünglich keine oder nur eine geringe Reverse Transkriptase-Aktivität aufweisen und durch Verwendung geeigneter Reaktionsbedingungen oder durch Mutationen als Reverse Transkriptase verwendet werden können (z.B. rTth-Polymerase).

Die wässrige Pufferlösung, in der die Degradierung von dsDNA in Gegenwart einer Reversen Transkriptase abläuft, enthält mindestens:
1) DNase I;
2) eine Reverse Transkriptase (wie oben beschrieben);
3) eine Puffersubstanz, die den pH-Wert des Versuchsansatzes puffert;
4) einen pH-Wert zwischen 6 und 10, besonders bevorzugt zwischen 7 und 9; und
5) bivalente Kationen, die eine Reverse Transkriptase-Reaktion und die enzymatische Degradierung von genomischer DNA unterstützen, wie z.B. Mg²⁺(in einem Konzentrationsbereich zwischen 0,1 und 50 mM), Mn²⁺ (in einem Konzentrationsbereich zwischen 0,01 und 10 mM), oder Ca²⁺ (in einem Konzentrationsbereich zwischen 0,01 und 50 mM).

Der Reaktionsansatz kann auch noch weitere Komponenten wie z.B. andere Enzyme, bivalente Kationen oder Salze enthalten. So kann z.B. auch eine hitzestabile DNA-Polymerase enthalten sein.

Die Reaktionstemperatur kann z.B. zwischen 10 und 70°C liegen, bevorzugt zwischen 15°C und 60°C, ganz besonders bevorzugt zwischen 20°C und 50°C.

Die Erfindung wird nachfolgend anhand der Beispiele näher erläutert.

### Beispiel 1

Jeweils 1 ng total-RNA aus HeLa-Zellen wurden mit 1 µg einer 0,2-9,5 kB RNA-Leiter (Invitrogen) gemischt, um in einer Reverse Transkriptase-Reaktion eingesetzt zu werden. Die Reverse Transkriptase-Reaktion wurde dabei in einem wässrigen Milieu durchgeführt, das einen Oligo-dT-Primer, dNTPs, RNase Inhibitor, einen Puffer (Buffer RT aus dem Omniscript RT Kit der QIAGEN GmbH, Hilden, Deutschland) für die reverse Transkription und eine Reverse Transkriptase (Omniscript^{®}, Marke der QIAGEN GmbH, Hilden, Deutschland) enthielt. Zudem wurde die DNA-Doppelstrang-spezifische Endonuklease DNase I (RNase-freie DNase I) zugegeben. In weiteren Ansätzen wurde Exonuklease VII ("Exo VII") hinzugefügt.

Die DNase I (aus Rinderpankreas; erhältlich von Roche, Mannheim, Deutschland) wie auch die Exonuclease VII wurde in unterschiedlichen Mengen verwendet. Einem weiteren Reaktionsansatz wurde keine Nuklease hinzugefügt. Dieser Ansatz diente als Positivkontrolle. Der Ansatz mit der Exonuclease VII diente als Negativkontrolle. Exonuklease VII ist nicht doppelstrangspezifisch und in der Lage, einzelsträngige DNA abzubauen. Die jeweiligen Reaktionsgemische wurden für 1 Stunde bei 37°C inkubiert und anschließend durch PCR auf cDNA-Degradierung analysiert. In der PCR wurde das gesamte Transkript von ß-Aktin amplifiziert.

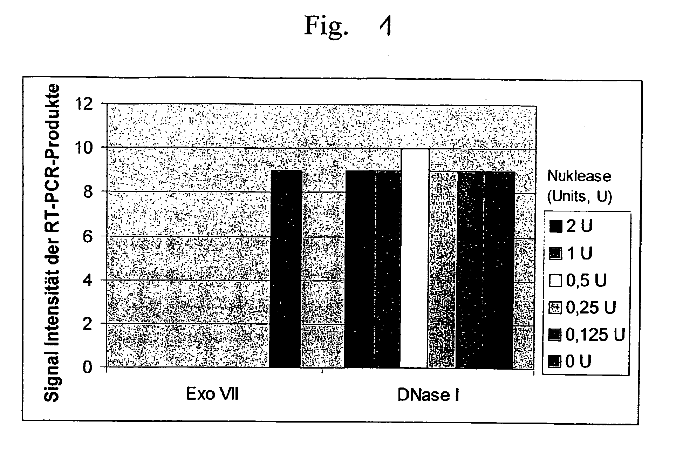

Das Ergebnis ist in Fig. 1 gezeigt. Die DNase I führte zu keiner Beeinträchtigung der Reverse Transkriptase-Reaktion. Dies ist ersichtlich gegenüber der Positivkontrolle DNase I zeigt eine Signalintensität an RT-PCR-Fragmenten, die der Intensität der Positivkontrolle entspricht. Nur die als Negativkontrolle verwendete einzelstrangspezifische Nuklease Exonuklease VII führte zu einer starken Degradierung der Einzelstrang-cDNA, so dass kein RT-PCR spezifisches Signal gefunden werden konnte.

### Beispiel 2

Jeweils 1 µg genomischer DNA und 1 µg Total-RNA aus HeLa-Zellen wurden gemischt, um in einer Reverse-Transkriptase Reaktion eingesetzt zu werden. Die Reverse Transkriptase-Reaktion wurde dabei in einem wässrigen Milieu durchgeführt, das einen Oligo-dT-Primer, dNTPs, RNase Inhibitor und einen Puffer (Buffer RT aus dem Omniscript RT Kit der QIAGEN GmbH, Hilden, Deutschland) für die reverse Transkription enthielt. Zudem wurden unterschiedliche Mengen an DNase I zugegeben:

### (1) RNase-freie DNase I in einer Menge von 0-10 U (Roche, Mannheim, Deutschland).

Einem Set der Ansätze wurde Reverse Transkriptase hinzugegeben, um den Einfluss der Nukleasen auf die Synthese einzelsträngiger cDNA untersuchen zu können. Einem zweiten Set von Ansätzen wurde keine reverse Transkriptase hinzugefügt, um die Degradierung der genomischen DNA verfolgen zu können. Die Reaktionsgemische wurden für 1 Stunde bei 37°C inkubiert und anschließend durch PCR analysiert. In den Ansätzen, in denen die cDNA-Synthese verfolgt wurde, wurde die vollständige cDNA des ß-Actin Transkriptes amplifiziert. Bei den Ansätzen, in denen die Degradierung der genomischen DNA verfolgt wurde, wurde ein Bereich aus dem 5'-Ende des ß-Actin Gens amplifiziert. Da das Primerset ein Intron überspannt, zeigt das genomische Amplifikat eine Größe von > 600 bp, während das Amplifikat der cDNA eine Größe von ca. 200 bp aufweist.

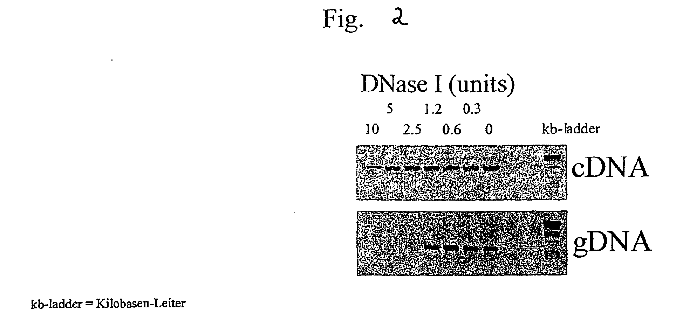

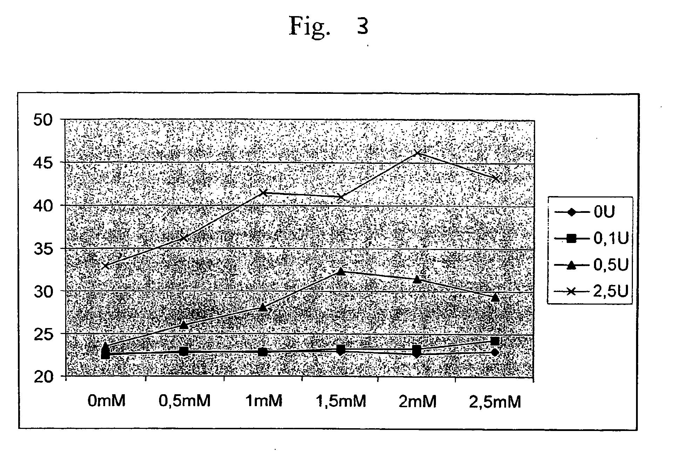

Das Ergebnis ist in Fig. 2 gezeigt. Figur 3 zeigt ein Foto eines 1%-igen Agarosegels auf dessen Bahnen Ansätze mit verschiedenen DNase I Konzentrationen untersucht wurden. Fig. 2 ist zu entnehmen, dass die reverse Transkription durch die Anwesenheit der getesteten DNase I nicht beeinträchtigt wird, was daran zu erkennen ist, dass die cDNA-Bande bei allen Konzentrationen klar sichtbar bleibt, siehe den oberen Bereich von Fig. 2. Dagegen ist ebenso klar erkennbar, dass die Verwendung der DNase I zu einer mehr oder weniger vollständigen Degradierung der eingesetzten genomischen DNA führt, wenn eine bestimmte Mindestmenge an Nuklease (5 U) zugesetzt wird, siehe unterer Bereich von Fig. 2.

### Beispiele 3

Jeweils 150 ng total-RNA aus HeLa-Zellen wurden mit 150 ng gDNA gemischt, um eine DNase-Reaktion unter Reverse Transkriptase Reaktionsbedingungen durchzuführen. Die Reaktion wurde dabei in einem wässrigen Milieu durchgeführt, das einen Oligo-dT-Primer, dNTPs, RNase Inhibitor, und einen Puffer (Buffer RT aus dem Omniscript RT Kit der QIAGEN GmbH, Hilden, Deutschland)_für die reverse Transkription enthielt. Zudem wurden zu den Ansätzen 0; 0,1; 0,5; oder 2,5 Units doppelstrangspezifische Nuklease zugegeben (RNase-freie DNase I). Zusätzlich wurden zu den Ansätzen 0 mM, 0,5 mM, 1 mM, 1,5 mM, 2 mM oder 2,5 mM Magnesiumchlorid hinzugefügt. Es wurde keine reverse Transkriptase hinzugegeben, um die DNase I Aktivität unter Reverse Transkriptase-Reaktionsbedingungen zu untersuchen Die Reaktionsgemische wurde für 1 Stunde bei 37°C inkubiert. Anschließend wurde der DNA-Abbau durch quantitative Echtzeit (Real-Time) PCR analysiert. Es wurden hierzu je 1 µl des Reaktionsgemisches für die Real-Time PCR eingesetzt. Hierbei wurde ein Primerpaar verwendet, das ein 200 bp Fragment aus dem 5'-Ende des β-Aktin amplifiziert. Das entstandene Amplifikat wurde durch SYBR Green detektiert.

Das Ergebnis ist in Fig. 3 und in Tabelle 1 gezeigt. Der Abbau der genomischen DNA wurde anhand des CT Wertes bestimmt und ist abhängig von der eingesetzten Menge RNase-freier DNase I. Durch Zugabe von zusätzlichem Magnesiumchlorid kann die Aktivität RNase-freier DNase I moduliert werden, wobei für 2,5 U DNase und 2 mM.MgCl₂ die höchsten CT-Werte erhalten wurden.

**Tabelle 1**

| MgCl₂ | DNase I (0 U) | DNase I (0,1 U) CT Cyclus | DNase I (0,5 U) CT Cyclus | DNase I (2,5 U) CT Cyclus |
|---|---|---|---|---|
| 0 mM | 22,7 | 22,5 | 23,5 | 32,9 |
| 0,5 mM | 22,8 | 22,9 | 26,1 | 36,2 |
| 1 mM | 22,9 | 22,8 | 28,1 | 41,5 |
| 1,5 mM | 22,9 | 23,3 | 32,3 | 41,1 |
| 2 mM | 22,6 | 23,3 | 31,4 | 46,2 |
| 2,5 mM | 22,9 | 24,3 | 29,4 | 43,2 |

### Beispiel 4

Jeweils 150 ng total-RNA aus HeLa-Zellen wurden mit 0 ng oder 150 ng gDNA und gemischt, um in einer Reverse Transkriptase-Reaktion eingesetzt zu werden. Die Reaktion fand dabei in einem wässrigen Milieu statt, das einen Oligo-dT-Primer, dNTPs, RNase Inhibitor und einen Puffer (Buffer RT aus dem Omniscript RT Kit der QIAGEN GmbH, Hilden, Deutschland) für die reverse Transkription enthielt. Zudem wurden zu einem Teil der Ansätze 2,5 Units doppelstrangspezifische Endonuklease zugegeben (RNase-freie DNase I). Einem Set der Ansätze wurde reverse Transkriptase hinzugegeben, um den Einfluss der Nukleasen auf die Synthese einzelsträngiger cDNA untersuchen zu können. Einem zweiten Set von Ansätzen wurde keine reverse Transkriptase hinzugegeben, um die Degradierung der genomischen DNA verfolgen zu können. Die Reaktionsgemische wurden für 1 Stunde bei 37°C inkubiert. Anschließend wurde die cDNA-Synthese und der DNA-Abbau durch quantitative Real-Time PCR analysiert. Es wurden je 1 µl und 0,1 µl der Reversen Transkriptase-Reaktion für die Real Time PCR eingesetzt. Hierbei wurde ein Primerpaar verwendet, das ein 210 bp Fragment aus dem 3'-Ende des β-Aktin amplifiziert. Das entstandene Amplifikat wurde durch SYBR Green detektiert.

Das Ergebnis dieses Versuches war, dass die reverse Transkription durch die Anwesenheit von RNase-freier DNase I nicht beeinträchtigt wird. Durch die Verwendung von RNase-freier DNase I wird die genomische DNA mehr als 1000-fach abgebaut. Gleichzeitig wird die generierte cDNA nicht oder nur unwesentlich verdaut. Das Ergebnis ist in Tabelle 2 zusammengefasst.

Der DNase-Schritt kann auch in einer sehr kurzen Reaktion vor der eigentlichen RNAverändernden Reaktion durchgeführt werden, wobei die DNase allerdings wie in den obigen Ansätzen in dem Reaktionsgemisch verbleibt und nicht durch Hitzeinaktivierung oder einen Aufreinigungsschritt aus dem System entfernt wird.

**Tabelle 2**

| **Übertragenes Volumen** | **CT Cyclus 150 ng Hela DNA** | **Mittelwert** | **CT Cyclus 0 ng Hela DNA** | **Mittelwert** |
|---|---|---|---|---|
| **Ohne reverse** | 22,1 | | 32,6 | |
| **Transkription,** | 22,4 | 22,2 | 33,5 | 33,4 |
| **ohne DNAse 1µl** | 22,0 | | 34,0 | |
| **Ohne reverse** | 32,9 | | 34,0 | |
| **Transkription, mit** | 31,8 | 32,2 | 35,2 | 33,5 |
| **DNAse 1µl** | 31,8 | | 31,3 | |
| **Ohne reverse** | 25,3 | | 34,4 | |
| **Transkription,** | 25,4 | 25,4 | 32,4 | 33,2 |
| **ohne DNAse 0,1µl** | 25,6 | | 32,9 | |
| **Ohne reverse** | 34,3 | | 32,6 | |
| **Transkription, mit** | 33,8 | 34,0 | 31,5 | 32,4 |
| **DNAse 0,1µl** | 33,9 | | 33,1 | |
| **Mit reverser** | 13,3 | | 13,4 | |
| **Transkription, mit** | 13,5 | 13,4 | 13,3 | 13,4 |
| **DNAse 1µl** | 13,4 | | 13,5 | |
| **Mit reverser** | 13,6 | | 13,8 | |
| **Transkription,** | 13,9 | 13,8 | 13,5 | 13,7 |
| **ohne DNAse 1µl** | 13,8 | | 13,7 | |
| **Mit reverser** | 16,3 | | 15,9 | |
| **Transkription, mit** | 16,5 | 16,5 | 16,4 | 16,3 |
| **DNAse 0,1µl** | 16,6 | | 16,7 | |
| **Mit reverser** | 16,0 | | 16,2 | |
| **Transkription,** | 16,5 | 16,3 | 16,6 | 16,5 |
| **ohne DNAse 0,1 µl** | 16,3 | | 16,6 | |

### Beispiel 5

Jeweils 10 pg bis 1 µg total-RNA aus HeLa-Zellen wurden mit identischen Mengen gDNA gemischt, um in einer Reverse Transkriptase-Reaktion eingesetzt zu werden. Die Reaktion fand in einem wässrigen Milieu statt, das einen Oligo-dT-Primer, random Oktamere, dNTPs, RNase Inhibitor und einen Puffer (gDNA Wipeout Buffer und Quantiscript RT Buffer aus dem QuantiTect® Reverse Transcription Kit der QIAGEN GmbH, Hilden, Deutschland) für die reverse Transkription enthielt. Zudem wurden zu einem Teil der Ansätze 2,5 Units doppelstrangspezifische Endonuklease zugegeben (RNase-freie DNase I). Einem Set der Ansätze wurde reverse Transkriptase hinzugefügt, um den Einfluss der Nukleasen auf die Synthese einzelsträngiger cDNA untersuchen zu können. Einem zweiten Set von Ansätzen wurde keine reverse Transkriptase hinzugegeben, um die Degradierung der genomischen DNA verfolgen zu können. Vor der eigentlichen cDNA Synthese wurde der DNase-Schritt für 2 min bei 37°C durchgeführt. Erst danach wurden die Reaktionsgemische für 15 min in Anwesenheit der Reversen Transkriptase bei 37°C inkubiert. Anschließend wurde die cDNA-Synthese und der DNA-Abbau durch quantitative Real-Time PCR analysiert. Es wurden je 1 µl der Reversen Transkriptase-Reaktion für die Real-Time PCR eingesetzt. Hierbei wurde ein QuantiTect Gene Expression Assay (QIAGEN GmbH, Hilden, Deutschland) für das Gen RPSLA verwendet, zusammen mit dem QuantiTect Probe PCR Kit (ebenfalls von QIAGEN) verwendet, der alle benötigten Reaktionskomponenten wie HotStar Taq DNA Polymerase (QIAGEN), Reaktionspuffer und dNTPs enthält. Die HotStar Taq DNA Polymerase wurde für 15 min bei 95°C reaktiviert, Darauf folgend wurden die PCR Reaktion für 50 Zyklen mit folgendem Temperaturprofil durchgeführt: 15 sec 56 °C, 30 sec 76°C, 30 sec 94°C. Vor dem Einsatz in der PCR-Reaktion wurde die Reverse Transkriptase-Reaktion für 5 min bei 95°C inaktiviert. Das Maß der genomischen DNA-Abreicherung wird in der folgenden Tabelle 3 in CT-Werten angegeben:

**Tabelle 3**

| Differenz -DNase/+DNase | | | | | | |
|---|---|---|---|---|---|---|
| | 1 µg | 100 ng | 10 ng | 1 ng | 100 pg | 10 pg |
| RNA 1 / DNA 1 | 20,6 | 18,7 | 18,1 | 17,8 | 14,2 | 5,4 |
| RNA 2 / DNA 2 | 23,3 | 20,7 | 20,0 | 13,6 | 8,3 | 10,4 |
| RNA 2 / DNA 1 | 23.1 | 21,7 | 21,4 | 18,8 | 14,8 | 12,1 |

Das Ergebnis zeigt, dass durch die Verwendung von RNase-freier DNase I, deren Inkubation dem eigentlichen Reverse Transkriptase-Schritt im Reaktionsansatz vorgeschaltet ist, die genomische DNA in der Regel mehr als 1000-fach abgereichert wird.

In einem weiteren Ansatz sollte gezeigt werden, dass der DNase-Schritt auch in den Prozess einer so genannten 1-Step-RT-PCR integriert werden kann. Bei einer 1-Step-RT-PCR Reaktion wird der gesamte Reaktionsansatz inklusive aller benötigten Reagenzien für den Reverse Transkriptase-Schritt und den anschließenden PCR Schritt zusammengefügt. Die Reaktion wird mit der Reversen Transkription gestartet und geht direkt ohne Öffnen des Reaktionsgefäßes in den PCR-Schritt über. Das folgende Beispiel zeigt, dass ein DNase-Schritt auch in ein solches durchgängiges Verfahrensschema, das keine weiteren Anwenderinteraktionen zulässt, eingefügt werden kann.

### Beispiel 6

Jeweils 20 ng total RNA aus HeLa Zellen und 20 ng hochmolekulare gDNA wurden in jeder 1-Step-RT-PCR-Reaktion eingesetzt. Der Reaktion wurden 150 µM CaCl₂ zugesetzt. Jede Reaktion wurde mit dem QuantiTect RT-PCR Kit (QIAGEN GmbH, Hilden, Deutschland) durchgeführt, welcher alle benötigten Reaktionskomponenten wie Reverse Transkriptase, HotStar Taq DNA Polymerase, Reaktionspuffer und dNTPs enthält. Die Reaktionen wurden mit und ohne DNase I angesetzt. In Reaktionen, die ausschließlich genomische DNA nachweisen sollten, wurde die Reverse Transkriptase nicht eingesetzt, um kein zusätzliches Signal von einer cDNA zu erhalten. Reaktionsansätze, in denen DNase I eingesetzt wurde, enthielten 0,25 Units DNase I. Als Zielgen wurde ein Transkriptbereich nachgewiesen, für das in der genomischen DNA identische Sequenzen vorkommen. Die PCR-Produkte, die aus genomischer DNA und cDNA generiert wurden, hatten die gleiche Größe und sollten demzufolge mit gleicher Effizienz amplifiziert und nachgewiesen werden.

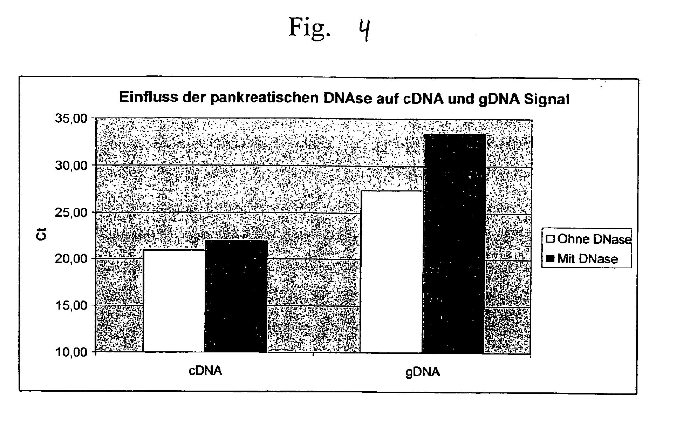

Das Ergebnis ist in Fig. 4 gezeigt und lässt sich wie folgt zusammenfassen. Der CT Wert der genomischen DNA vergrößert sich durch Einsatz der DNase um mehr als 6 Zyklen, was einem hundertfachen gDNA-Abbau entspricht, während sich der CT Wert der cDNA nur unwesentlich verändert. Dies lässt die Schlussfolgerung zu, dass ein gDNA-Entfemungsschritt auch in einer 1-Step-RT-PCR einsetzbar ist und zu einem signifikanten Abbau der genomischen DNA führt, während die cDNA intakt bleibt oder nur unwesentlich degradiert wird. Die Erhöhung des CT Wertes bei Verwendung von RNA (cDNA) ist im Wesentlichen auf die Abreicherung der in der RNA Probe enthaltenen genomischen DNA zurückzuführen.

## Patentansprüche

1. RNA-Reaktionsverfahren, **dadurch gekennzeichnet, dass** in demselben Reaktionsgefäß zur gleichen Zeit und bei derselben Temperatur eine RNA-Reaktion und eine Degradierung von vorhandener doppelsträngiger DNA erfolgen, wobei die Degradierung der doppelsträngigen DNA durch DNase I erfolgt und wobei die RNA-Reaktion eine Umsetzung ist, bei der RNA als Matrize für Polymerasereaktionen verwendet wird und in einzelsträngige DNA umgeschrieben wird.

2. RNA-Reaktionsverfahren nach Anspruch 1, **dadurch gekennzeichnet, dass** die Umsetzung eine reverse Transkription ist.

3. RNA-Reaktionsverfahren nach einem der Ansprüche 1 bis 2, **dadurch gekennzeichnet, dass** die Reaktion bei einem pH-Wert zwischen 6 und 10, vorzugsweise zwischen 7 und 9, durchgeführt wird.

4. RNA-Reaktionsverfahren nach einem der Ansprüche 1 bis 3, **dadurch gekennzeichnet, dass** der Reaktionsansatz bivalente Kationen enthält.

5. RNA-Reaktionsverfahren nach Anspruch 4, **dadurch gekennzeichnet, dass** die bivalenten Kationen Mg²⁺, Mn²⁺ und/oder Ca²⁺ sind.

## Claims

1. RNA reaction method, **characterized in that** an RNA reaction and a degradation of existing doublestranded DNA are carried out in the same reaction vessel at the same time and at the same temperature, the degradation of the doublestranded DNA being effected by DNase I and the RNA reaction being a conversion in which RNA is used as template for polymerase reactions and is transcribed into single-stranded DNA.

2. RNA reaction method according to Claim 1, **characterized in that** the conversion is a reverse transcription.

3. RNA reaction method according to any of Claims 1 to 2, **characterized in that** the reaction is carried out at a pH of between 6 and 10, preferably between 7 and 9.

4. RNA reaction method according to any of Claims 1 to 3, **characterized in that** the reaction mixture contains bivalent cations.

5. RNA reaction method according to Claim 4, **characterized in that** the bivalent cations are Mg²⁺, Mn²⁺ and/or Ca²⁺.

## Revendications

1. Procédé de réaction de l'ARN, **caractérisé en ce qu'**on procède simultanément, dans le même réacteur et à la même température, à une réaction de l'ARN et à une dégradation de l'ADN double brin présent, la dégradation de l'ADN double brin ayant lieu grâce à l'ADN-ase I, la réaction de l'ARN étant une réaction lors de laquelle l'ARN est utilisé sous forme de matrice pour des réactions par polymérase, et est transcrit en un ADN simple brin.

2. Procédé de réaction de l'ARN selon la revendication 1, **caractérisé en ce que** la réaction est une transcription inverse.

3. Procédé de réaction de l'ARN selon l'une des revendications 1 à 2, **caractérisé en ce que** la réaction est mise en oeuvre à un pH compris entre 6 et 10, de préférence entre 7 et 9.

4. Procédé de réaction de l'ARN selon l'une des revendications 1 à 3, **caractérisé en ce que** la masse réactionnelle contient des cations bivalents.

5. Procédé de réaction de l'ARN selon la revendication 4, **caractérisé en ce que** les cations bivalents sont Mg²⁺, Mn²⁺ et/ou Ca²⁺.
